# EUROPEAN PATENT APPLICATION

(11) **EP 3 249 399 A1**
(43) Date of publication of application: **29.11.2017**
(21) Application number: 17177836.8
(22) Date of filing: 22.09.2012
(51) Int. Cl.: G01N 33/48, G01N 33/574, A61B 5/145

(54) **DETECTING OVARIAN CANCER**

(30) Priority: 22.09.2011 US 201161626202 P
(62) Divisional of application: 12834127.8
(71) Applicant: Memorial Sloan-Kettering Cancer Center, New York, NY 10065 (US)
(72) Inventor: LEVINE, Douglas A., New York, NY 10065 (US)
(74) Representative: Schiweck, Weinzierl & Koch Patentanwälte Partnerschaft mbB

(57) **Abstract**

Among other things, the present disclosure provides a method including the steps of: obtaining a uterine sample; and detecting and/or characterizing in the uterine sample an ovarian cancer biomarker (e.g., CA125).

## Description

### Related Cases

This application claims priority to United States provisional patent application serial number 61/626,202, filed September 22, 2011 and titled "Methods and Devices for the Early Detection of Ovarian Cancer," the entire contents of which are herein incorporated by reference.

### Background

Ovarian cancer is the leading cause of gynecologic cancer death and the fifth most common cause of cancer death in American women. Although steady increases in survival duration have been achieved, cure rates for advanced disease have been stubbornly low. Stage for stage, ovarian cancer has similar survival rates to most other solid tumors. However, most ovarian cancer presents at an advanced stage unlike colon, breast, and prostate cancers where reliable screening and early detection methods exist. Most ovarian cancer presents at an advanced state due to the particular difficulty in detecting ovarian cancer in the earlier stages. In particular there is a lack of reliable screening and detection methods for ovarian cancer in the earlier stages.

### Summary

The present invention provides technologies for detecting and/or characterizing cancer, and in particular ovarian cancer, which is well known to be one of the most difficult cancers to detect. For example, the present invention encompasses the surprising finding that ovarian washes can contain material and/or markers that permit detection and/or characterization of cancer, and particularly of ovarian cancer, including ovarian cancer that arises or exists in fallopian tubes. The present invention also encompasses the surprising finding that certain devices (e.g., so-called "nanogetter" devices) are particularly useful in the detection of ovarian cancer, for example in intrauterine fluid or uterine washes.

In accordance with some embodiments of the present invention, a method includes the steps of: obtaining a uterine sample; and measuring in the uterine sample an ovarian cancer biomarker level.

In accordance with some embodiments, the ovarian may be CA-125.

In accordance with some embodiments, the ovarian cancer biomarker may be selected from the group consisting of HE4, mesothelin, and YKL-40.

In accordance with some embodiments, the step of obtaining may include obtaining a uterine wash sample.

In accordance with some embodiments, the step of obtaining may include obtaining a sample in vivo.

In accordance with some embodiments, the step of measuring occurs in vivo.

In accordance with some embodiments, the step of obtaining and the step of measuring may be performed via a bio-sensing device implanted in vivo.

In accordance with some embodiments, the method may further include transmitting a signal from the bio-sensing device to a remote sensor, the signal corresponding to data obtained during the step of measuring.

In accordance with some embodiments, the method may further include adjusting a therapy based on data obtained during the step of measuring.

In accordance with some embodiments, the method may further include repeatedly performing the step of measuring.

In accordance with some embodiments, the method may further include adjusting a therapy based on data obtained during the step of repeatedly performing the step of measuring.

In accordance with some embodiments, the step of measuring may include determining that the biomarker is present at a level higher than a reference level.

In accordance with some embodiments, the reference level may be correlated with presence or absence of cancer.

In accordance with some embodiments, the reference level may be correlated with absence or presence of cancer at a particular stage.

In accordance with some embodiments, the step of measuring may include determining that the biomarker is present at a level lower than a reference level.

In accordance with some embodiments, the reference level may be correlated with presence or absence of cancer.

In accordance with some embodiments, the reference level may be correlated with absence or presence of cancer at a particular stage.

In accordance with some embodiments, the step of measuring may include determining that the biomarker is present at a level comparable to a reference level.

In accordance with some embodiments, the reference level may be correlated with presence or absence of cancer.

In accordance with some embodiments, the reference level may be correlated with absence or presence of cancer at a particular stage.

In accordance with some embodiments of the present invention, a method includes steps of detecting an ovarian cancer biomarker in situ in a mammalian subject's endometrial/uterine cavity.

Further features and aspects of example embodiments of the present invention are described in more detail below with reference to the appended Figures.

### Brief Description of the Drawing

**Fig. 1** shows a model of ovarian carcinogenesis.

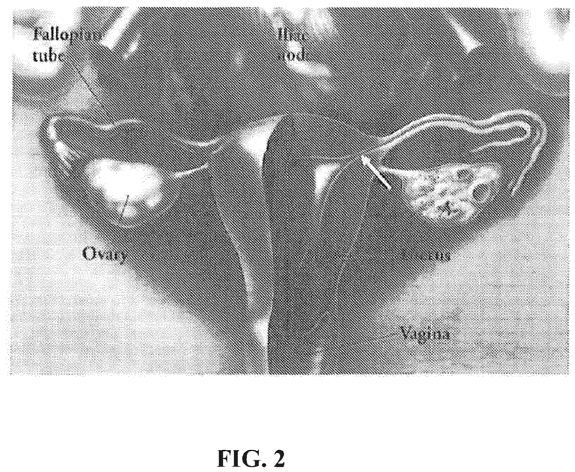

**Fig. 2** shows an anatomic figure demonstrating the continuity between the fallopian tubes and the uterine cavity, which is indicated by a superimposed arrow.

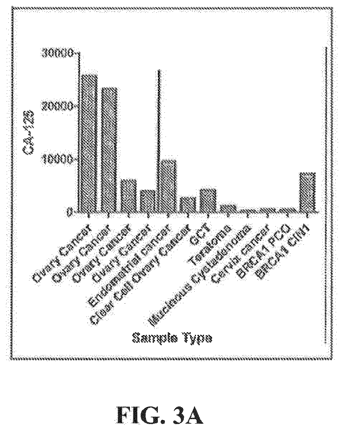

**Fig. 3A** shows CA125 levels in uterine washings.

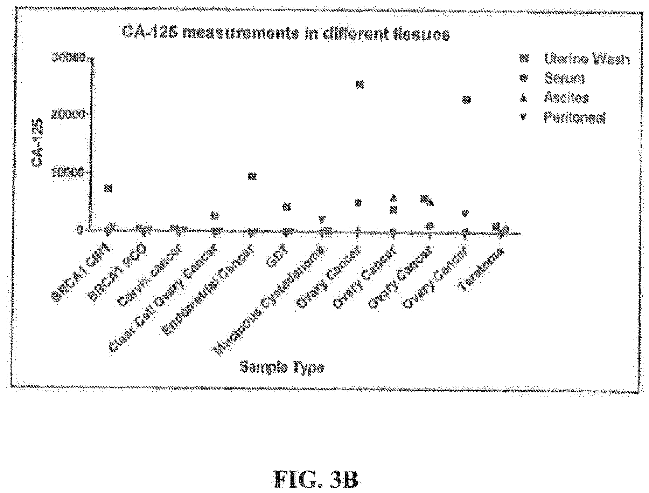

**Fig. 3B** shows CA125 levels in various tissues.
**Fig. 4** shows a bio-sensor.
**Fig. 5** shows levels of ovarian biomarkers in uterine washes.

### Definitions

In order for the present disclosure to be more readily understood, certain terms are first defined below. Additional definitions for the following terms and other terms are set forth throughout the specification.

***Adjust:*** As used herein when referring to therapies, the term "adjust" means to change. In some embodiments, "adjusting" is or comprises initiating. In some embodiments, "adjusting" is or comprises terminating. In some embodiments, "adjusting" is or comprises changing one or more features or aspects relative to a reference therapy.

***Biomarker:*** The term "biomarker" as used herein has its art-understood meaning of an agent or entity whose presence or level correlates with an event of interest. In some embodiments, the event of interest is presence or type of a particular cell or tissue (e.g., ovarian cells and/or ovarian cancer). In some embodiments, a particular agent or entity correlates with an event of interest if it is statistically more likely to be present (or present at a particular level) when the event occurs or is present than under otherwise comparable conditions absent the event. In some embodiments, a particular agent or entity correlates with an event of interest if it is present at a level at least 1.5, 2, 2.5, 3, 3.5, 4, 4.5, 5, 6, 7, 8, 9, 10, 15, 20, 25, 30, 35, 40, 45, 50, 60, 70, 80, 90, 200 fold or more higher when the event occurs or is present than under otherwise comparable conditions absent the event.

***Combination therapy:*** As used herein, the term "combination therapy", as used herein, refers to those situations in which two or more different pharmaceutical agents or therapeutic modalities are administered in overlapping regimens so that the subject is simultaneously exposed to both agents.

***Comparable:*** The term "comparable" as used herein refers to a system, set of conditions, effects, or results that is/are sufficiently similar to a reference system, set of conditions, effects, or results, to permit scientifically legitimate comparison. Those of ordinary skill in the art will appreciate and understand which systems, sets of conditions, effect, or results are sufficiently similar to be "comparable" to any particular reference system, set of conditions, effects, or results as described herein.

***Dosage form:*** As used herein, the terms "dosage form" and "unit dosage form" refer to a physically discrete unit of a therapeutic agent for the patient to be treated. Each unit contains a predetermined quantity of active material calculated to produce the desired therapeutic effect. It will be understood, however, that the total dosage of the composition will be decided by the attending physician within the scope of sound medical judgment.

***Dosing regimen:*** A "dosing regimen" (or "therapeutic regimen"), as that term is used herein, is a set of unit doses (typically more than one) that are administered individually to a subject, typically separated by periods of time. In some embodiments, a given therapeutic agent has a recommended dosing regimen, which may involve one or more doses. In some embodiments, a dosing regimen comprises a plurality of doses each of which are separated from one another by a time period of the same length; in some embodiments, a dosing regimen comprises a plurality of doses and at least two different time periods separating individual doses. In some embodiments, the therapeutic agent is administered continuously over a predetermined period.

***Ovarian cancer:*** As used herein, the term "ovarian cancer" refers to any cancer of Mullerian origin, which includes traditionally diagnosed ovarian, fallopian tube and primary peritoneal cancers. In some embodiments, ovarian cancer is any high-grade serous carcinoma of Mullerian origin which includes traditionally diagnosed ovarian, fallopian tube and primary peritoneal cancers. The National Cancer Institute of the United States (NCI) defines ovarian cancer as "Cancer that forms in tissues of the ovary (one of a pair of female reproductive glands in which the ova, or eggs, are formed). Most ovarian cancers are either ovarian epithelial carcinomas (cancer that begins in the cells on the surface of the ovary) or malignant germ cell tumors (cancer that begins in egg cells)."

***Reference Therapy:*** In some embodiments, the term "reference means a standard therapy (see Example 4).

***Sample:*** The term "sample", as used herein, refers to a composition containing one or more materials from a source. In some embodiments, the source is or comprises a tissue, cell, or organism. In some embodiments, a sample is a primary sample of such a tissue, cell, or organism. Is some embodiments, a sample is obtained by processing a primary sample, for example to remove one or more components and/or to enrich or purify one or more components. In some embodiments, a sample is a wash preparation; in some embodiments, a sample is prepared from a wash, for example, by removal of one or more components and/or enrichment or purification of one or more components.

***Wash preparation:*** A wash preparation is a liquid composition that contains one or more components of a cell, tissue or organism. In some embodiments, a wash is prepared by contacting a washing fluid with a cell, tissue or organism under conditions and for a period of time sufficient to permit certain components of a cell, tissue or organism to enter the washing fluid so that a wash preparation is generated. In some such embodiments, the washing fluid is contacted with the cell, tissue or organism under such conditions and for such time that no significant cell lysis occurs; rather, cells and/or tissues remain substantially intact. In certain such embodiments, the wash preparation contains cells, cellular fragments, proteins and/or nucleic acids. In some embodiments the proteins contained in the wash preparation are detectable by ELISA assays. You could easily detect DNA and we have other ongoing studies that do this in different experimental systems. In some embodiments, the wash preparation contains cells, cellular fragments, proteins and/or nucleic acids arising in the ovary or fallopian tube.

### Detailed Description of Certain Embodiments

The present disclosure provides, among other things, technologies for detecting cancer in uterine samples and/or in situ.

### Ovarian Cancer

Ovarian cancer is the leading cause of gynecologic cancer death and the fifth most common cause of cancer death in American women. Although steady increases in survival duration have been achieved, cure rates for advanced disease have been stubbornly low. Stage for stage, ovarian cancer has similar survival rates to most other solid tumors. However, most ovarian cancer presents at an advanced stage unlike colon, breast, and prostate cancers where reliable screening and early detection methods exist.

For more than a decade ultrasonography and serum CA-125 measurements have been tested alone or in various combinations in order to detect early stage ovarian carcinoma. Most of these studies have been unsuccessful and when early stage cancer has been detected it most often has been non-serous, less aggressive, less common histologic subtypes. Recent reports have confirmed the inability to detect early stage ovarian cancer with combined or sequential sonography and CA-125. The screening studies have also found that the number of diagnostic surgical procedures is too great when compared to the number of early tumors identified; with the possible exception of the ongoing UKCTOCS which has shown some promise in its preliminary analyses.

Due to the general and relatively consistent failure of screening studies using ultrasonography and serum CA-125 measurements, new and innovative approaches to early detection of ovarian cancer are clearly needed.

One of the limitations to successful early detection has been an inadequate understanding of the early events of ovarian carcinogenesis. Unfortunately, unlike cancers arising in the colon, breast, cervix, endometrium, prostate, and pancreas, where the early events of carcinogenesis can be studied because their precursor lesions have been well recognized, the precursor of ovarian carcinoma has eluded detection.

The teaching for decades is that ovarian carcinoma originates in the ovarian surface epithelium (OSE) or ovarian cortical inclusion cysts (CICs) but these reports are descriptive studies containing a very small number of cases and therefore the prevalence of these lesions is unknown. Over many years of examining gynecologic specimens, thousands of ovaries have been reviewed, including those from women with BRCA mutations, many containing multiple CICs, but lesions resembling an intraepithelial carcinoma in CICs and the OSE have been identified in only a few instances. In contrast, a recently described putative precursor of ovarian carcinoma has been described in the distal fallopian tube, designated "serous tubal intraepithelial carcinoma (STIC)".

STIC lesions are found in association with "ovarian" high-grade serous carcinomas (HGSCs) in 50-60% of cases and in the fallopian tubes removed along with ovaries prophylactically in approximately 15% of high-risk women. Accordingly, it is believed that HGSC is likely to arise in general from precursor tubal lesions which are much more common and, unlike the changes in CICs and OSE, have a morphologic, immunohistochemical (IHC) and molecular genetic phenotype closely resembling ovarian HGSC.

The dualistic model of ovarian carcinogenesis proposed by Kurman RJ, Shih IM, Molecular pathogenesis and extraovarian origin of epithelial ovarian cancer-shifting the paradigm. Hum Pathol. 2011 Jul; 42(7): 918-31 (hereinafter "Kurman and Shih") (hereby incorporated by reference in its entirety), divides ovarian carcinomas into two broad categories (type I and type II) based on morphologic, molecular genetic and clinical features and draws attention to the heterogeneity of these carcinomas and their specific precursors underscoring the importance of segregating different histologic subtypes when trying to elucidate their histogenesis. Thus, STICs are precursors of HGSC (type II tumor), whereas endometriosis is a precursor of endometrioid and clear cell carcinoma; and borderline tumors are precursors of low-grade serous and mucinous carcinomas (type I tumors). The model not only highlights the distinctive morphologic, molecular genetic and clinical characteristics that link the precursor lesions to specific histologic subtypes, it also points out the need to target efforts on the type II tumors of which HGSC is the prototype. HGSC represents 75% of all ovarian cancers and accounts for 90% of the deaths.

Accordingly, it is an object of example embodiments of the present invention to focus on the early events associated with HGSC, as it clearly is the most important histologic subtype in terms of frequency and mortality.

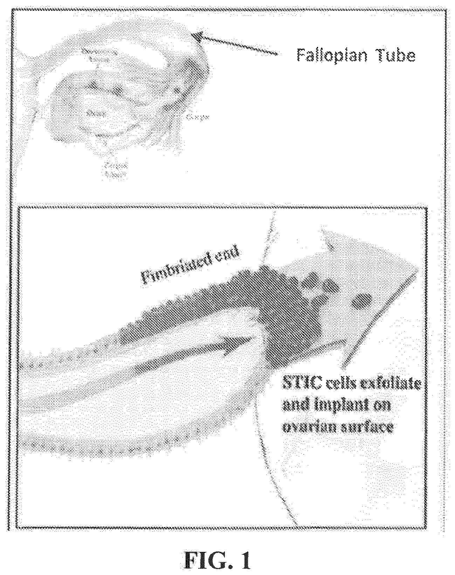

**Fig. 1****,** taken from Kurman and Shih and adapted, illustrates the implantation of STIC cells from the fimbria to the ovarian surface. Without wishing to be bound by any particular theory, the present inventors propose that STIC is the precursor of many, if not most, HGSCs.

Clear cell, endometrioid and mucinous carcinomas are clinically important but they represent only 25 % of all ovarian carcinomas and account for 10% of deaths. In contrast, as noted above, HGSC represents 75% of all ovarian cancers and accounts for 90% of the deaths. Accordingly, our goals focus exclusively on the early events associated with HGSC, as it clearly is the most important histologic subtype in terms of frequency and mortality.

As noted above, the OSE and ovarian CICs have been considered the leading precursor candidates but their occurrence as reported in the literature and in our experience is quite uncommon. A significant shortcoming of studies describing precursor lesions involving the OSE immediately adjacent to HGSC is that they have not taken into account that the "precursor" lesion is not a precursor, but instead represents lateral spread of carcinoma on the OSE. Another shortcoming is that none of these studies sampled the fallopian tubes and therefore did not exclude the possibility that the ovarian lesions are not metastases from an occult primary carcinoma in fallopian tube. These shortcomings are significant in light of recent data implicating the possible tubal origin of ovarian carcinomas. Specifically, morphologic, IHC, and molecular genetic evidence has indicated that when the fallopian tubes are completely examined, microscopically STICs, which display morphologic and molecular genetic features that closely resemble ovarian HGSC, are frequently found in association with ovarian HGSC. It has been proposed that cells from STICs are shed from the fallopian tube and implant on the ovary, developing into a tumor, which simulates a primary ovarian HGSC (**Fig**. **1****).** Two important findings from the published literature emerge: 1) that a lesion resembling ovarian HGSC is rarely reported in CICs and OSE compared to STICs and 2) that there is evidence for and against the role of CICs and OSE as precursors whereas all the papers describing STICs support that it is a precursor lesion.

Papers describing STICS include, for example:
Crum CP, Drapkin R, Miron A, Ince TA, Muto M, Kindelberger DW, Lee Y. The distal fallopian tube: a new model for pelvic serous carcinogenesis. Curr Opin Obstet Gynecol 12007;19: 3-9, the entire contents of which are herein incorporated by reference;
Kindelberger DW, Lee Y, Miron A, Hirsch MS, Feltmate C, Medeiros F, Callahan MJ, Garner EO, Gordon RW, Birch C, Berkowitz RS, Muto MG, Crum CP. Intraepithelial carcinoma of the fimbria and pelvic serous carcinoma: Evidence for a causal relationship. Am J Surg Pathol 12007;31: 161-9, the entire contents of which are herein incorporated by reference;
Kuhn E, Kurman RJ, Vang R, Sehdev AS, Han G, Soslow R, Wang TL, and Shih IeM. TP53 mutations in serous tubal intraepithelial carcinoma and concurrent high-grade serous carcinoma-evidence supporting the clonal relationship of the two lesions. J Pathol 2012;226:421-6, the entire contents of which are herein incorporated by reference;
Leeper K, Garcia R, Swisher E, Goff B, Greer B, Paley P. Pathologic findings in prophylactic oophorectomy specimens in high-risk women. Gynecol Oncol 12002;87: 52-6, the entire contents of which are herein incorporated by reference;
Levanon K, Crum CP, Drapkin R. New insights into the pathogenesis of serous ovarian cancer and its clinical impact. J Clin Oncol 2008;26: 5284-93, the entire contents of which are herein incorporated by reference;
Medeiros F, Muto MG, Lee Y, Elvin JA, Callahan MJ, Feltmate C, Garber JE, Cramer DW, Crum CP. The tubal fimbria is a preferred site for early adenocarcinoma in women with familial ovarian cancer syndrome. Am J Surg Pathol 12006;30: 230-6, the entire contents of which are herein incorporated by reference; and
Przybycin CG, Kurman RJ, Ronnett BM, Shih Ie M, Vang R. Are all pelvic (nonuterine) serous carcinomas of tubal origin? Am J Surg Pathol 12010;34: 1407-16, the entire contents of which are herein incorporated by reference.

### Origin of ovarian cancer

The cell of origin of ovarian cancer has been long debated. The traditional view has been that epithelial ovarian tumors are derived from the OSE. Unfortunately, despite diligent searches over the last 30 years, a widely accepted precursor in these locations has been only rarely reported. Moreover, unlike the epithelium of the cervix, endometrium and fallopian tubes, the epithelium covering the ovaries is not derived from the mullerian ducts but rather from mesodermal epithelium on the urogenital ridge. A recent molecular genetic analysis showed aneuploidy in CICs but not the OSE supporting the proposal that ovarian carcinoma begins in these cysts rather than the OSE. It is speculated, however, that CICs may not develop from the OSE but from FTE. In hindsight, it is believed that the failure to identify the tubal carcinomas in the past was because it was assumed that precursors of ovarian carcinoma would logically be in the ovaries, and therefore the fallopian tubes were not carefully examined. It was subsequently proposed that implantation of malignant cells from the tubal carcinoma to the ovary develop into a tumor mass that gives the impression that the tumor originated in the ovary.

Additional studies in which fallopian tubes were carefully examined confirmed that STICs and small, early invasive tubal carcinomas occurred not only in women with a genetic predisposition for the development of ovarian cancer but also in 50-60% of women without known BRCA mutations (sporadic ovarian cancer). Moreover, these carcinomas were almost always detected in the fimbria. Further evidence supporting the proposal that STICs are precursors was the identification of STICs in women without ovarian cancer as well as the presence of identical TP53 mutations in STICs and concomitant ovarian HGSCs, indicating a clonal relationship between them. Additional evidence implicating the FTE rather than OSE as the site of origin of HGSC comes from a gene-profiling study showing that the gene expression profile of HGSC is more closely related to FTE than to OSE. Immunohistochemical studies showing that HGSC expresses PAX8, a mullerian marker, but not calretinin, a mesothelial marker (OSE has a mesothelial not a mullerian morphologic phenotype).

### CA-125

The circulating CA125 antigen measured in the serum has been a mainstay of ovarian cancer assessment and management over the past 25 years. The cloning of CA125, achieved in 2001, first identified MUC 16 as a tethered mucin with a small intracellular domain, a transmembrane domain, an ectodomain proximal to the putative cleavage site and a large, heavily glycosylated region of 12-20 tandem repeats. Molecular identification has provided new opportunities to explore MUC 16 biology and pathogenic behaviors of this large protein and its contribution to ovarian biology. Compared to other mucins, the expression of MUC16 is more restricted and is confined, almost exclusively to the mullerian tract, even in malignancy. Interestingly, MUC16 is also present in the ocular epithelium. Although MUC16 has very limited expression in normal human tissues, it is present in the vast majority of serous ovarian cancers. The MUC 16 molecule appears to function as a key interacting protein with mesothelin and other stromal proteins, and may explain some of the unique biological behaviors of serous ovarian cancer. In clinical settings, high levels of the circulating peptides from MUC16 which encode or carry the CA125 antigen are associated with an adverse clinical outcome, independent of stage, grade and other traditional clinical factors. Amplification of genomic regions encoding MUC16 in ovarian cancer DNA has been observed in The Cancer Genome Atlas ovarian cancer project. Moreover, as described herein, artificial MUC16 protein has been transfected into four MUC16 negative cell lines. Results suggest that expression of this protein is associated with specific alterations of signal transduction, gene expression and aggressive biological behavior.

In view of the aforementioned understanding of the origins of ovarian cancer in the fallopian tube coupled with the importance of CA125 in ovarian cancer biology, example embodiments of the present invention employ strategies to increase the likelihood of early detection of ovarian cancer.

While stage I ovarian cancer has a 90% cure rate, over three quarters of patients are diagnosed with advanced disease where the cure rates still hover near 25%. Serum markers like MUC16/CA-125 have been used for the detection of ovarian carcinoma for decades, but have failed in the ability to detect early stage ovarian carcinoma through serum measurements. Recent studies have demonstrated that the majority of presumed ovarian carcinoma likely develops within the fallopian tube. The fallopian tube lies in direct continuity with the endometrial/uterine cavity, which can serve as a proximal anatomic site for the detection of tissue specific biomarkers (**Fig**. **2****).** Furthermore, nanotechnology creates the opportunity to change the entire paradigm of ovarian cancer screening using long-term monitoring devices within appropriate body cavities.

### Uterine Sample

The present disclosure surprisingly demonstrates that CA-125 measurements obtained from uterine washings and/or in situ can be used to identify and/or characterize individuals suffering from ovarian carcinoma or known precursor lesion such as, for example, STIC. This approach may be adapted to the office setting similar to an endometrial biopsy, which is commonly performed. This novel approach to early ovarian cancer detection capitalizes on recent knowledge that the putative cell of origin for ovarian cancer is the fallopian tubal secretory epithelium and not the ovarian surface epithelium, as had been thought for decades.

### Markers

In addition or as an alternative to CA-125, other biomarkers, e.g., other biomarkers detectable in uterine washings and/or in situ as described herein, may be measured to identify patients with ovarian carcinoma. Those of ordinary skill in the art are familiar with a variety of biomarkers whose presence, and/or level correlates with presence and/or type of ovarian cells and/or with ovarian cancer. To give but a few examples, biomarkers such as HE4, mesothelin, and/or YKL-40 may be detected and/or measured.

In some embodiments, a biomarker detected and/or measured as described herein is an ovarian biomarker in that it is statistically more likely to be associated with ovarian cells than non-ovarian cells. In some embodiments, a biomarker detected and/or measured as described herein is a cancer biomarker in that its presence or level is statistically more likely to be associated with cancer cells than will non-cancer cells. In some embodiments, a biomarker detected and/or measured as described herein is an ovarian cancer biomarker in that its presence or level is statistically more likely to be associated with ovarian cancer cells than with one or more reference cell types. In some embodiments, the reference cell type is non-cancerous cells. In some embodiments, the reference cell type is non-cancerous ovarian cells.

In some embodiments, a biomarker associated with particular cells or types of cells is present in or on those cells or cell types at a level at least 1.5, 2, 2.5, 3, 3.5, 4, 4.5, 5, 6, 7, 8, 9, 10, 15, 20, 25, 30, 35, 40, 45, 50, 60, 70, 80, 90, 200 fold or more higher than it is in or on reference cells or cell types.

### Detection Systems

In some embodiments nanotechnology may be employed for remote bio-sensing of cancer antigens. In some embodiments a bio-sensing device may be configured to intermittently and frequently monitor body tissues for appropriate molecules and/or proteins. Such a device could, for example, be implantable into various body cavities. In some embodiments, such a bio-sensing device may include a microfluidics concentrator chamber, a transducer layer and a transceiver layer. In some such embodiments, body fluids pass through the microfluidics concentrator chamber and proteins are detected on the transducer layer. When appropriate molecules are generated a signal is created that is transmitted from the transceiver layer to activate a remote sensor.

In some embodiments, the transducer is functionalized for detection of one or more relevant biomarkers. In some such embodiments, such functionalization comprises association of one or more antibodies to the biomarker(s) with the transducer layer. To give but one specific example, in some embodiments, antibodies are coupled to a dendrimer backbone that when separated activates carbon nanotubes to create an electric signal detected by the transceiver layer. Example 2 describes design and assembly of one example of such a device.

In some embodiments, useful devices in accordance with the present invention include multiple antibodies applied to a solid support structure such that molecules may be detected in nano-molar range. In some embodiments, such devices allow for repeated, frequent measurements such that interval development of disease is limited.

### Applications

In some embodiments, methods and/or devices in accordance with the invention are used to detect and/or characterize ovarian cancer even during early stages.

In some embodiments, the stage of the ovarian cancer may be determined. For example, the measured level of one or more biomarker-taken alone and/or in comparison to the measured level of one or more other biomarkers-may be analyzed and/or correlated to a particular level of ovarian cancer. Alternatively or in addition, the measured level of one or more biomarker (absolute and/or relative) may be compared to prior and/or subsequent corresponding measurements taken at, for example, predetermined time intervals.

In some embodiments, the biomarker measurement or measurements may be analyzed to determine an adjustment of a therapy for treatment of ovarian cancer and/or to determine whether or not any adjustment is needed. For example, a reference dosage or dosage regime may be adjusted based on the measurements. In some embodiments, the measurements are taken periodically over a period of time. In some embodiments, the periodically taken measurements are monitored and/or analyzed to periodically and/or continually adjust the therapy. In some embodiments, the periodically taken measurements are monitored and/or analyzed to monitor the progress and/or effectiveness of the therapy.

In some embodiments, one or biomarker measurement may by utilized to predict a therapy. For example, a set of periodic measurements may be analyzed to predict a therapy that is expected to be effective based on the set of measurements. In some embodiments, the periodic measurements may be analyzed to determine the patient's responsiveness to a prior therapy and, based on the periodic measurements and the characteristics of the prior therapy, determine an ongoing therapy that is predicted to be effective. In some embodiments, the determined ongoing therapy may reflect a change in the dosage or dosage regime of one or more drugs used in the prior therapy and/or a change in the type of drug or drug combination utilized in the prior therapy.

In some embodiments, the present invention includes a step of measuring a level of one or more biomarkers. In some such embodiments, such measuring determines that the measured level is above a threshold or reference level. In some embodiments, such measuring determines that the measured level is below a threshold or reference level. In some embodiments, such measuring determines that the measured level is comparable to a threshold or reference level.

In some embodiments, a determination that the measured level is different from (i.e., above or below) the threshold or reference level constitutes a determination that ovarian cancer is likely to be present and/or to be at a particular stage. In some embodiments, a determination that the measured level is comparable to a threshold or reference level constitutes a determination that ovarian cancer is likely or unlikely to be present and/or to be at a particular stage.

In some embodiments, a threshold or reference level is that observed in non-ovarian cells. In some embodiments, a threshold or reference level is that observed in non-cancerous cells. In some embodiments, a threshold or reference level is that observed in known cancer cells. In some embodiments, a threshold or reference level is that observed in cancer cells of a known stage. In some embodiments, the cancer cells are ovarian cancer cells.

In some embodiments, the measurement of biomarker level and comparison of this measured level with a reference level results in a determination of presence, absence, or stage of ovarian cancer. In some such embodiments, this determination warrants a change in therapy.

To give but a few examples, in some embodiments, a biomarker level is measured in accordance with the present invention to be higher than a reference level in non-cancerous ovarian cells and a determination is made that ovarian cancer is present. In some such embodiments, the measured level is comparable to that correlated with presence of ovarian cancer of a particular stage, and a determination is made that the ovarian cancer is at the relevant stage. In some embodiments, the measured level is below that correlated with presence of ovarian cancer of a particular stage, and a determination is made that the ovarian cancer has not reached the relevant stage.

Those of skill in the art will well appreciate the various determinations that are appropriately made as a result of comparisons between measured levels and threshold reference levels, and also of steps appropriately taken in light of such determinations. In some embodiments, instructions are given to take such steps and/or such steps are performed.

In some embodiments, a threshold or reference level is determined substantially simultaneously with the biomarker measurement. In some embodiments, a threshold or reference level is determined at a different time. For example, in some embodiments, a threshold or reference level is a historical threshold or reference level, or otherwise is a level known to be correlated with a particular event of interest (e.g., presence or stage of ovarian cells, and in particular ovarian cancer cells).

The invention is further characterized by following items.
Item 1. A method comprising steps of: obtaining a uterine sample; and measuring in the uterine sample an ovarian cancer biomarker level.
Item 2. The method of item 1, wherein the ovarian cancer biomarker is CA-125.
Item 3. The method of item 1, wherein the ovarian cancer biomarker is selected from the group consisting of HE4, mesothelin, and YKL-40.
Item 4. The method of item 1, wherein the step of obtaining comprises obtaining a uterine wash sample.
Item 5. The method of item 1, wherein the step of obtaining comprises obtaining a sample in vivo.
Item 6. The method of item 5, wherein the step of measuring occurs in vivo.
Item 7. The method of item 6, wherein the step of obtaining and the step of measuring are performed via a bio-sensing device implanted in vivo.
Item 8. The method of item 7, further comprising transmitting a signal from the bio-sensing device to a remote sensor, the signal corresponding to data obtained during the step of measuring.
Item 9. The method of item 1, further comprising adjusting a therapy based on data obtained during the step of measuring.
Item 10. The method of item 1, further comprising repeatedly performing the step of measuring.
Item 11. The method of item 10, further comprising adjusting a therapy based on data obtained during the step of repeatedly performing the step of measuring.
Item 12. The method of item 1, wherein the step of measuring comprises determining that the biomarker is present at a level higher than a reference level.
Item 13. The method of item 12, wherein the reference level is correlated with presence or absence of cancer.
Item 14. The method of item 12, wherein the reference level is correlated with absence or presence of cancer at a particular stage.
Item 15. The method of item 1, wherein the step of measuring comprises determining that the biomarker is present at a level lower than a reference level.
Item 16. The method of item 15, wherein the reference level is correlated with presence or absence of cancer.
Item 17. The method of item 15, wherein the reference level is correlated with absence or presence of cancer at a particular stage.
Item 18. The method of item 1, wherein the step of measuring comprises determining that the biomarker is present at a level comparable to a reference level.
Item 19. The method of item 18, wherein the reference level is correlated with presence or absence of cancer.
Item 20. The method of item 18, wherein the reference level is correlated with absence or presence of cancer at a particular stage.
Item 21. A method comprising steps of detecting an ovarian cancer biomarker in situ in a mammalian subject's endometrial/uterine cavity.

### Exemplification

Aspects of the present disclosure may be further understood in light of the following examples, which are not exhaustive and which should not be construed as limiting the scope of the inventions described here in any way.

### Example 1: Uterine Washes

The present Example describes certain exemplary methodologies for obtaining uterine washes.

In accordance with example embodiments, uterine washings are obtained by placing a uterine manipulator into the patient's uterus after first dilating the uterine cervix. Approximately 20-40cc of normal saline is infused into the uterine cavity for example though an access port in a uterine manipulator. The normal saline is then collected from the posterior fornix of the vagina where it pools. The uterine manipulator is left in place for the remainder of the case to be used as clinically indicated or removed immediately. While in the operating room, a separate 5cc sample of peripheral blood is drawn to measure serum CA-125. Upon entering the abdomen for ovarian cancer surgery, risk-reducing salpingo-oophorectomy, or other gynecologic procedure, 20-40cc of ascites is aspirated for CA-125 measurements, if present. If ascites is not present, peritoneal washings are obtained as per standard clinical protocols. For each abdominal specimen type, ascites or peritoneal washings, a specimen is obtained for clinical diagnostic purposes and a second specimen may be obtained for research purposes. Abdominal specimens are obtained through an open incision or laparoscopically, as dictated by clinical circumstances. Serum CA-125 values are run in real-time and measurements from other body tissues are batched weekly.

### Example 2: Design and Assembly of a Nanogetter Device Utilizing Dendrimer -Coupled Anti-CA-125/MUC16 Antibodies

The present Example describes design and construction of an exemplary "Nanogetter" device that utilizes dendrimer-coupled anti-CA-125/MUC16 antibodies.

Initially, several different CA-125/MUC16 antibodies will be coupled to the dendrimer backbone to ensure reliable linkage. This construct will then be applied to single or multi-walled carbon nanotubes. We will determine that the carbon nanotubes can detect various concentrations of MUC16. We will then apply the device in vitro to ovarian cancer cells to detect the protein within a biologically relevant background.

A "Nanogetter" device as described in U.S. Patent Application Publication No. 2009/0220382, which is hereby incorporated in its entirety by reference thereto, has previously been shown to be highly sensitive, able to detect parts per billion within a "cluttered" background.

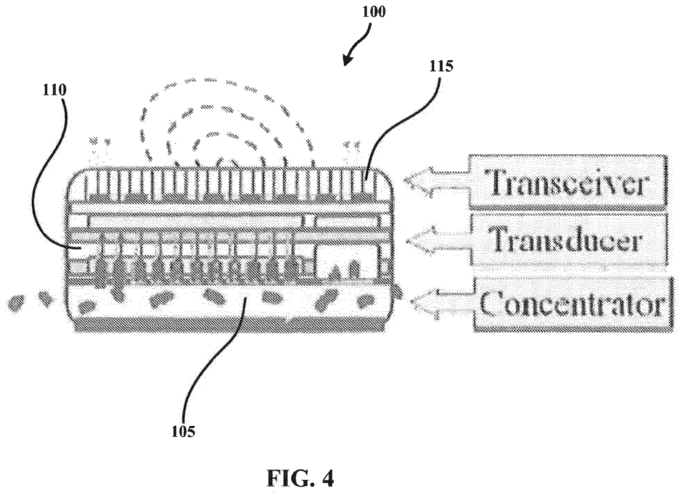

Referring to **Fig. 4****,** an example bio-sensing device 100 is configured to intermittently and frequently monitor body tissues for appropriate molecules and/or proteins. The device 100 is implantable into various body cavities. The bio-sensing device 100 includes a microfluidics concentrator chamber 105, a transducer layer 110 and a transceiver layer 115. Body fluids pass through the micro fluidics concentrator chamber 105 and proteins are detected on the transducer layer 110. When appropriate molecules are generated a signal is created that is transmitted from the transceiver layer 115 to activate a remote sensor.

### Example 3: Uterine Washing Biomarkers as a Novel Screening Tool for High-Grade Serous Carcinoma

This Example describes clinical studies demonstrating that analysis of appropriate biomarkers in uterine washes can identify women with serous carcinoma and can distinguish them from women with benign conditions of the ovary.

**Objective:** High-grade serous carcinoma (HGSC) of the pelvis is an aggressive gynecologic malignancy often detected at advanced stage. Clinically proven screening tests are lacking. Recent data suggest that the precursor lesion - serous tubal intraepithelial carcinoma - arises from the distal fallopian tube. Without wishing to be bound by any particular theory, present inventors propose that given HGSC's tubal origin, biomarkers previously studied in the serum may also be elevated in washings from the uterine cavity, representing a novel screening approach to early detection.

**Methods:** Women undergoing primary cytoreduction for stage IIIC or IV high-grade serous carcinoma and those undergoing bilateral salpingo-oophorectomy (BSO) for benign indications were enrolled. Patients with concurrent malignancy, prior malignancy requiring pelvic radiation, and previous hysterectomy were excluded. Serum, uterine washings, and peritoneal washings were collected from each patient and analyzed for CA-125, YKL-40, HE4 and mesothelin (MSLN).

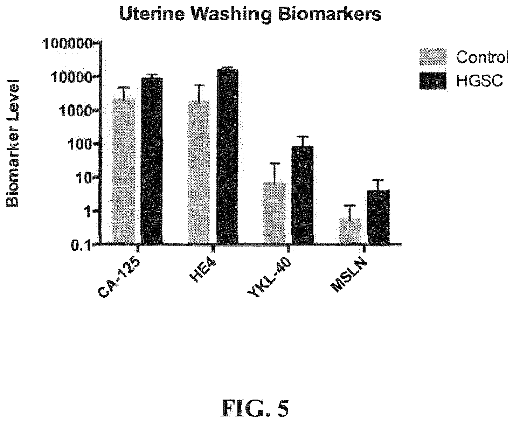

**Results:** Of twenty-five women studied, 11 had high-grade serous carcinoma and 14 were non-cancer controls. Age and BRCA status were similar in the two groups (*P >* 0.05 for both). As shown in Fig. **5****,** CA-125, YKL-40, HE4 and MSLN levels in serum, peritoneal washings, and uterine washings were all significantly higher in HGSC patients than patients with benign histology. In uterine washing specimens, median CA125 values were 4.4-fold greater in carcinoma patients than in controls (8520U/mL vs. 1955U/mL, *P =* 0.008). Median YKL-40, HE4, and MSLN uterine washing levels were 6 to 12-fold greater in carcinoma patients than in controls (P < 0.004 for all, see Figure). Furthermore, median values for CA125 and HE4 in cancer patients were 38 and 23-fold higher in the uterine washings than serum, respectively. YKL-40 levels in cancer patients were ∼50% lower in uterine washings than serum and MSLN levels were similar. ROC curves were constructed for each biomarker, and the AUC for both serum and uterine washings was > 0.8 for all four markers, suggesting that elevation is highly associated with disease status.

**Conclusions:** Common ovarian biomarkers are elevated in the uterine washings from HGSC patients in this pilot study. Though this study was restricted to advanced stage HGSC patients, it provides a foundation to explore a new approach to ovarian cancer screening considering a putative precursor lesion within the distal fallopian tube.

### Example 4: Standard Therapy for Ovarian Cancer

The National Cancer Institute lists several drugs as approved therapies for ovarian cancer. For each, at least one standard formulation and therapeutic regimen has been approved, as set out below. Each such standard formulation and therapeutic regimen can be considered a "reference" therapeutic regimen for purposes of the present disclosure. http://www.cancer.gov/cancertopics/druginfo/doxorubicinhydrochloride

### ACDIAMYCIN RDF/PFS (DOXYRUBICIN HYDROCHLORIDE)

### DESCRIPTION

Doxorubicin is a cytotoxic anthracycline antibiotic isolated from cultures of Streptomyces peucetius var. caesius.

Doxorubicin consists of a naphthacenequinone nucleus linked through a glycosidic bond at ring atom 7 to an amino sugar, daunosamine.

Chemically, doxorubicin hydrochloride is: 5,12-Naphthacenedione, 10-[(3-amino-2,3,6-trideoxy-alpha-L-lyxo-hexopyranosyl)oxy]-7,8,9,10-tetrahydro-6,8,11-trihydroxy-8-(hydroxylacetyl)-1-methoxy-, hydrochloride (8S-cis)-. The structural formula is as follows:

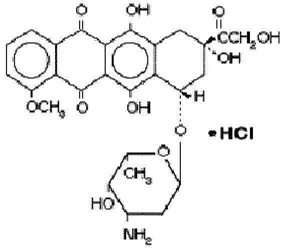

ADRIAMYCIN RDF® (Doxorubicin Hydrochloride for Injection, USP) a sterile red-orange lyophilized powder for intravenous use only, is available in 10, 20 and 50 mg single dose vials and a 150 mg multidose vial.

Each 10 mg single dose vial contains 10 mg of doxorubicin HCl, USP, 50 mg of lactose, NF (hydrous) and 1 mg of methylparaben, NF (added to enhance dissolution) as a sterile red-orange lyophilized powder.

Each 20 mg single dose vial contains 20 mg of doxorubicin HCl, USP, 100 mg of lactose, NF (hydrous) and 2 mg of methylparaben, NF (added to enhance dissolution) as a sterile red-orange lyophilized powder.

Each 50 mg single dose vial contains 50 mg of doxorubicin HCl, USP, 250 mg of lactose, NF (hydrous) and 5 mg of methylparaben, NF (added to enhance dissolution) as a sterile red-orange lyophilized powder.

Each 150 mg multidose vial contains 150 mg of doxorubicin HCl, USP, 750 mg of lactose, NF (hydrous) and 15 mg of methylparaben, NF (added to enhance dissolution) as a sterile red-orange lyophilized powder.

ADRIAMYCIN PFS® (Doxorubicin Hydrochloride Injection, USP) is a sterile parenteral, isotonic solution for intravenous use only, containing no preservative, available in 5 mL (10 mg), 10 mL (20 mg), 25 mL (50 mg), and 37.5 mL (75 mg) single dose vials and a 100 mL (200 mg) multidose vial.

Each mL contains doxorubicin HCI 2 mg, USP and the following inactive ingredients: sodium chloride 0.9% and water for injection q.s. Hydrochloric acid is used to adjust the pH to a target pH of 3.0.

### INDICATIONS AND USAGE

ADRIAMYCIN PFS and ADRIAMYCIN RDF have been used successfully to produce regression in disseminated neoplastic conditions such as acute lymphoblastic leukemia, acute myeloblastic leukemia, Wilms' tumor, neuroblastoma, soft tissue and bone sarcomas, breast carcinoma, ovarian carcinoma, transitional cell bladder carcinoma, thyroid carcinoma, gastric carcinoma, Hodgkin's disease, malignant lymphoma and bronchogenic carcinoma in which the small cell histologic type is the most responsive compared to other cell types.

### DOSAGE AND ADMINISTRATION

The most commonly used dose schedule when used as a single agent is 60 to 75 mg/m² as a single intravenous injection administered at 21-day intervals. The lower dosage should be given to patients with inadequate marrow reserves due to old age, or prior therapy, or neoplastic marrow infiltration. ADRIAMYCIN PFS and ADRIAMYCIN RDF have been used concurrently with other approved chemotherapeutic agents. Evidence is available that in some types of neoplastic disease combination chemotherapy is superior to single agents. The benefits and risks of such therapy continue to be elucidated. When used in combination with other chemotherapy drugs, the most commonly used dosage of doxorubicin is 40 to 60 mg/m² given as a single intravenous injection every 21 to 28 days. Doxorubicin dosage must be reduced in case of hyperbilirubinemia as follows:

| **Plasma bilirubin concentration (mg/dL)** | **Dosage reduction (%)** |
|---|---|
| 1.2 - 3.0 | 50 |
| 3.1 - 5.0 | 75 |

### CARBOPLATIN

### DESCRIPTION

Carboplatin Injection is supplied as a sterile, pyrogen-free, 10 mg/mL aqueous solution of Carboplatin. Carboplatin is a platinum coordination compound. The chemical name for carboplatin is platinum, diammine[1,1-cyclobutane-dicarboxylato(2-)-O,O']-,(SP-4-2), and carboplatin has the following structural formula:

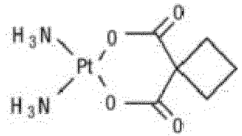

Carboplatin is a crystalline powder with the molecular formula of C6H12N2O4Pt and a molecular weight of 371.25. It is soluble in water at a rate of approximately 14 mg/mL, and the pH of a 1% solution is 5-7. It is virtually insoluble in ethanol, acetone, and dimethylacetamide.

Each ml of carboplatin injection contains 10 mg of carboplatin in water for injection, with no other inactive ingredients, pH range is 5.0-7.0. It is supplied as 5 ml, 15 ml & 45 ml multi dose vials.

### INDICATIONS AND USAGE

### Initial Treatment of Advanced Ovarian Carcinoma

Carboplatin Injection is indicated for the initial treatment of advanced ovarian carcinoma in established combination with other approved chemotherapeutic agents. One established combination regimen consists of carboplatin and cyclophosphamide. Two randomized controlled studies conducted by the NCIC and SWOG with carboplatin vs. cisplatin, both in combination with cyclophosphamide, have demonstrated equivalent overall survival between the two groups.

There is limited statistical power to demonstrate equivalence in overall pathologic complete response rates and long-term survival (≥ 3 years) because of the small number of patients with these outcomes: the small number of patients with residual tumor <2 cm after initial surgery also limits the statistical power to demonstrate equivalence in this subgroup.

### Secondary Treatment of Advanced Ovarian Carcinoma

Carboplatin is indicated for the palliative treatment of patients with ovarian carcinoma recurrent after prior chemotherapy, including patients who have been previously treated with cisplatin.

Within the group of patients previously treated with cisplatin, those who have developed progressive disease while receiving cisplatin therapy may have a decreased response rate.

### DOSAGE AND ADMINISTRATION

### Single-Agent Therapy

Carboplatin Injection, as a single agent, has been shown to be effective in patients with recurrent ovarian carcinoma at a dosage of 360 mg/m2 IV on day 1 every 4 weeks (alternatively see Formula Dosing, below). In general, however, single intermittent courses of carboplatin injection should not be repeated until the neutrophil count is at least 2000 and the platelet count is at least 100,000.

### Combination Therapy with Cyclophosphamide

In the chemotherapy of advanced ovarian cancer, an effective combination for previously untreated patients consists of :
Carboplatin Injection -300 mg/m2 IV on day 1 every four weeks for six cycles (alternatively see Formula Dosing, below).
Cyclophosphamide-600 mg/m2 IV on day 1 every four weeks for six cycles.

Intermittent courses of carboplatin Injection in combination with cyclophosphamide should not be repeated until the neutrophil count is at least 2000 and the platelet count is at least 100,000.

### Dose Adjustment

Pretreatment platelet count and performance status are important prognostic factors for severity of myelosuppression in previously treated patients.

The suggested dose adjustments for single agent or combination therapy shown in the table below are modified from controlled trials in previously treated and untreated patients with ovarian carcinoma. Blood counts were done weekly, and the recommendations are based on the lowest post-treatment platelet or neutrophil value.

| **Platelets** | **Neutrophils** | **Adjusted Dose * (From Prior Course)** |
|---|---|---|
| >100,000 | >2000 | 125% |
| 50-100,000 | 500-2000 | No Adjustment |
| <50,000 | <500 | 75% |

| | | |
|---|---|---|
| * Percentages apply to carboplatin Injection as a single agent or to both carboplatin Injection and cyclophosphamide in combination. In the controlled studies, dosages were also adjusted at a lower level (50 to 60%) for severe myelosuppression. Escalations above 125% were not recommended for these studies. | | |

Carboplatin Injection is usually administered by an infusion lasting 15 minutes or longer. No pre- or post-treatment hydration or forced diuresis is required.

### Patients with Impaired Kidney Function

Patients with creatinine clearance values below 60 mL/min are at increased risk of severe bone marrow suppression. In renally-impaired patients who received single-agent carboplatin Injection therapy, the incidence of severe leukopenia, neutropenia, or thrombocytopenia has been about 25% when the dosage modifications in the table below have been used.

| **Baseline Creatinine Clearance** | **Recommended Dose on Day 1** |
|---|---|
| 41-59 mL/min | 250 mg/m² |
| 16-40 mL/min | 200 mg/m² |

The data available for patients with severely impaired kidney function (creatinine clearance below 15 mL/min) are too limited to permit a recommendation for treatment.

These dosing recommendations apply to the initial course of treatment. Subsequent dosages should be adjusted according to the patient's tolerance based on the degree of bone marrow suppression.

### Formula Dosing

Another approach for determining the initial dose of carboplatin Injection is the use of mathematical formulae, which are based on a patient's pre-existing renal function or renal function and desired platelet nadir. Renal excretion is the major route of elimination for carboplatin. The use of dosing formulae, as compared to empirical dose calculation based on body surface area, allows compensation for patient variations in pretreatment renal function that might otherwise result in either underdosing (in patients with above average renal function) or overdosing (in patients with impaired renal function).

A simple formula for calculating dosage, based upon a patient's glomerular filtration rate (GFR in mL/min) and carboplatin target area under the concentration versus time curve (AUC in mg/mL•min), has been proposed by Calvert. In these studies, GFR was measured by 51Cr-EDTA clearance.

| CALVERT FORMULA FOR CARBOPLATIN INJECTION DOUSING |
|---|
| Total Dose (mg) = (target AUC) × (GFR + 25) |
| **Note: With the Calvert formula, the total dose of carboplatin Injection is calculated in mg, not mg/m².** |

The target AUC of 4-6 mg/mL•min using single-agent carboplatin Injection appears to provide the most appropriate dose range in previously treated patients. This study also showed a trend between the AUC of single-agent carboplatin Injection administered to previously treated patients and the likelihood of developing toxicity.

| | % Actual Toxicity in Previously Treated Patients | |
|---|---|---|
| AUC (mg/mL•min) | Gr 3 or Gr 4 Thrombocytopenia | Gr 3 or Gr 4 Leukopenia |
| 4 to 5 | 16% | 13% |
| 6 to 7 | 33% | 34% |

### Geriatric Dosing

Because renal function is often decreased in elderly patients, formula dosing of carboplatin Injection based on estimates of GFR should be used in elderly patients to provide predictable plasma carboplatin AUCs and thereby minimize the risk of toxicity.

### CLAFEN (CYCLOPHOSPHAMIDE)

### DESCRIPTION

Cyclophosphamide is a synthetic antineoplastic drug chemically related to the nitrogen mustards. Cyclophosphamide is a white crystalline powder with the molecular formula C7H15C12N2O2P • H2O and a molecular weight of 279.1. The chemical name for cyclophosphamide is 2H-1,3,2-Oxazaphosphorin-2-amine, N,N-bis(2-chloroethyl)tetrahydro-, 2-oxide, monohydrate, (±). Cyclophosphamide is soluble in water, saline, or ethanol and has the following structural formula:

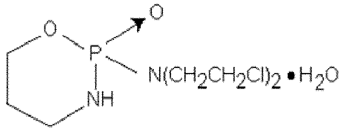

Each tablet for oral administration contains cyclophosphamide (anhydrous) 25 mg or 50 mg. In addition, each tablet contains the following inactive ingredients: acacia, FD&C Blue No. 1, lactose monohydrate, magnesium stearate, and microcrystalline cellulose.

### INDICATIONS AND USAGE

Cyclophosphamide Tablets USP, 25 mg and 50 mg, although effective alone in susceptible malignancies, are more frequently used concurrently or sequentially with other antineoplastic drugs. Adenocarcinoma of the ovary is often susceptible to cyclophosphamide treatment.

### DOSAGE AND ADMINISTRATION

For Treatment of Malignant Diseases in Adults and Children: Oral cyclophosphamide dosing is usually in the range of 1 to 5 mg/kg/day for both initial and maintenance dosing.

Many other regimens of intravenous and oral cyclophosphamide have been reported. Dosages must be adjusted in accord with evidence of antitumor activity and/or leukopenia. The total leukocyte count is a good, objective guide for regulating dosage. Transient decreases in the total white blood cell count to 2000 cells/mm3 (following short courses) or more persistent reduction to 3000 cells/mm3 (with continuing therapy) are tolerated without serious risk of infection if there is no marked granulocytopenia.

When cyclophosphamide is included in combined cytotoxic regimens, it may be necessary to reduce the dose of cyclophosphamide, as well as that of the other drugs.

Cyclophosphamide and its metabolites are dialyzable although there are probably quantitative differences depending upon the dialysis system being used. Patients with compromised renal function may show some measurable changes in pharmacokinetic parameters of cyclophosphamide metabolism, but there is no consistent evidence indicating a need for cyclophosphamide dosage modification in patients with renal function impairment.

### Cisplatin

### DESCRIPTION

PLATINOL® (cisplatin for injection, USP) is a white to light yellow lyophilized powder. Each vial of PLATINOL contains 50 mg cisplatin, 450 mg Sodium Chloride, USP, and 500 mg Mannitol, USP.

The active ingredient, cisplatin, is a yellow to orange crystalline powder with the molecular formula PtCl2H6N2, and a molecular weight of 300.1. Cisplatin is a heavy metal complex containing a central atom of platinum surrounded by two chloride atoms and two ammonia molecules in the cis position. It is soluble in water or saline at 1 mg/mL and in dimethylformamide at 24 mg/mL. It has a melting point of 207° C.

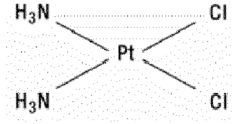

### INDICATIONS AND USAGE

PLATINOL (cisplatin for injection, USP) is indicated as therapy to be employed as follows:
Metastatic Ovarian Tumors

In established combination therapy with other approved chemotherapeutic agents in patients with metastatic ovarian tumors who have already received appropriate surgical and/or radiotherapeutic procedures. An established combination consists of PLATINOL and cyclophosphamide. PLATINOL, as a single agent, is indicated as secondary therapy in patients with metastatic ovarian tumors refractory to standard chemotherapy who have not previously received PLATINOL therapy.

### DOSAGE AND ADMINISTRATION

PLATINOL is administered by slow intravenous infusion. Platinol should not be given by rapid intravenous injection.

The usual PLATINOL dose for the treatment of metastatic ovarian tumors in combination with cyclophosphamide is 75 to 100 mg/m2 IV per cycle once every 4 weeks (DAY 1).

The dose of cyclophosphamide when used in combination with PLATINOL is 600 mg/m2 IV once every 4 weeks (DAY 1). In combination therapy, PLATINOL and cyclophosphamide are administered sequentially.

As a single agent, PLATINOL should be administered at a dose of 100 mg/m2 IV per cycle once every 4 weeks.

Pretreatment hydration with 1 to 2 liters of fluid infused for 8 to 12 hours prior to a PLATINOL dose is recommended. The drug is then diluted in 2 liters of 5% Dextrose in 1/2 or 1/3 normal saline containing 37.5 g of mannitol, and infused over a 6- to 8-hour period. If diluted solution is not to be used within 6 hours, protect solution from light. Adequate hydration and urinary output must be maintained during the following 24 hours.

A repeat course of PLATINOL should not be given until the serum creatinine is below 1.5 mg/100 mL, and/or the BUN is below 25 mg/100 mL. A repeat course should not be given until circulating blood elements are at an acceptable level (platelets >100,000/mm3, WBC ≥4000/mm3). Subsequent doses of PLATINOL should not be given until an audiometric analysis indicates that auditory acuity is within normal limits.

### Doxorubicin Hydrochloride

### DESCRIPTION

Doxorubicin is a cytotoxic anthracycline antibiotic isolated from cultures of Streptomyces peucetius var. caesius. Doxorubicin consists of a naphthacenequinone nucleus linked through a glycosidic bond at ring atom 7 to an amino sugar, daunosamine. Chemically, doxorubicin hydrochloride is: 5,12-Naphthacenedione, 10-[(3-amino-2,3,6-trideoxy-α-L-lyxohexopyranosyl)oxy]-7,8,9,10-tetrahydro-6,8,11-trihydroxy-8-(hydroxylacetyl)-1-methoxy-, hydrochloride (8S-cis)-. The structural formula is as follows:

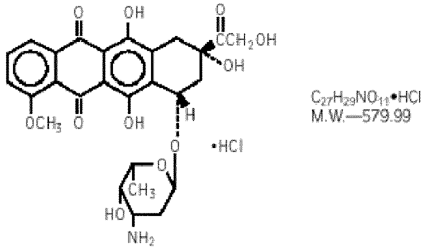

Doxorubicin binds to nucleic acids, presumably by specific intercalation of the planar anthracycline nucleus with the DNA double helix. The anthracycline ring is lipophilic, but the saturated end of the ring system contains abundant hydroxyl groups adjacent to the amino sugar, producing a hydrophilic center. The molecule is amphoteric, containing acidic functions in the ring phenolic groups and a basic function in the sugar amino group. It binds to cell membranes as well as plasma proteins.

Doxorubicin Hydrochloride for Injection, USP is a sterile red-orange lyophilized powder for intravenous use only. Doxorubicin Hydrochloride for Injection, USP is available in 10, 20, and 50 mg single dose vials and a 150 mg multidose vial. Each single dose vial contains 10, 20, and 50 mg of doxorubicin HCl, USP. Each 150 mg multidose vial contains 150 mg of doxorubicin HCl, USP. Inactive ingredients include lactose, NF (hydrous), and methylparaben, NF.

Doxorubicin Hydrochloride Injection, USP is a clear, red, sterile, isotonic aqueous solution. Doxorubicin Hydrochloride Injection, USP is packaged in either single dose polypropylene vials containing 10, 20, and 50 mg of doxorubicin HCl, USP, or multidose vials containing 150 and 200 mg doxorubicin HCl, USP, as preservative-free, ready-to-use solution. Each milliliter of solution contains 2 mg of doxorubicin HCl. Inactive ingredients include sodium chloride 0.9%, USP, and water for injection, USP, q.s. The pH of the solution has been adjusted to 3.0 with hydrochloric acid, USP.

### INDICATIONS AND USAGE

Doxorubicin has been used successfully to produce regression in disseminated neoplastic conditions such as acute lymphoblastic leukemia, acute myeloblastic leukemia, Wilms' tumor, neuroblastoma, soft tissue and bone sarcomas, breast carcinoma, ovarian carcinoma, transitional cell bladder carcinoma, thyroid carcinoma, gastric carcinoma, Hodgkin's disease, malignant lymphoma, and bronchogenic carcinoma in which the small cell histologic type is the most responsive compared to other cell types.

### DOSAGE AND ADMINISTRATION

The most commonly used dose schedule when used as a single agent is 60 to 75 mg/m2 as a single intravenous injection administered at 21-day intervals. The lower dosage should be given to patients with inadequate marrow reserves due to old age, or prior therapy, or neoplastic marrow infiltration.

Doxorubicin has been used concurrently with other approved chemotherapeutic agents. Evidence is available that in some types of neoplastic disease, combination chemotherapy is superior to single agents. The benefits and risks of such therapy continue to be elucidated. When used in combination with other chemotherapy drugs, the most commonly used dosage of doxorubicin is 40 to 60 mg/m2 given as a single intravenous injection every 21 to 28 days.

Doxorubicin dosage must be reduced in case of hyperbilirubinemia as follows:

| **Plasma bilirubin concentration (mg/dL)** | **Dosage reduction (%)** |
|---|---|
| 1.2 - 3.0 | 50 |
| 3.1 *-* 5.0 | 75 |

### DOXIL (Doxorubicin Hydrochloride Liposome)

### DESCRIPTION

Doxil® (doxorubicin HCl liposome injection) is doxorubicin hydrochloride (HCl) encapsulated in STEALTH® liposomes for intravenous administration.

Doxorubicin is a cytotoxic anthracycline antibiotic isolated from Streptomyces peucetius var. caesius.

Doxorubicin HCl, which is the established name for (8S,10S)-10-[(3-amino-2,3,6-trideoxy-a-L-lyxo-hexopyranosyl)oxy]-8-glycolyl-7,8,9,10-tetrahydro-6,8,11-trihydroxy-1-methoxy-5,12-naphthacenedione hydrochloride, has the following structure:

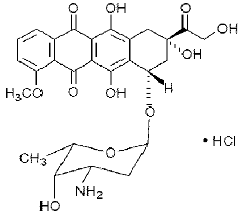

The molecular formula of the drug is C27 H29 NO11·HCl; its molecular weight is 579.99. Doxil® is provided as a sterile, translucent, red liposomal dispersion in 10-mL

glass, single use vials. Each vial contains 20 mg doxorubicin HCl at a concentration of 2 mg/mL and a pH of 6.5. The STEALTH® liposome carriers are composed of N-(carbonyl-methoxypolyethylene glycol 2000)-1,2-distearoyl-snglycero-3-phosphoethanolamine sodium salt (MPEG-DSPE), 3.19 mg/mL; fully hydrogenated soy phosphatidylcholine (HSPC), 9.58 mg/mL; and cholesterol, 3.19 mg/mL. Each mL also contains ammonium sulfate, approximately 2 mg; histidine as a buffer; hydrochloric acid and/or sodium hydroxide for pH control; and sucrose to maintain isotonicity. Greater than 90% of the drug is encapsulated in the STEALTH® liposomes.

MPEG-DSPE has the following structural formula:

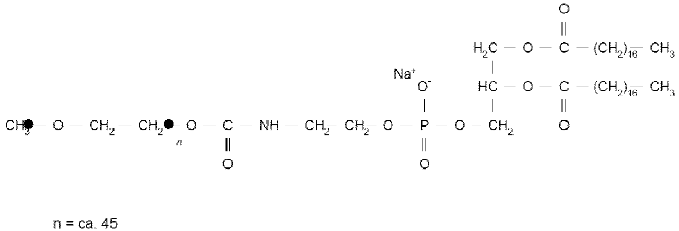

HSPC has the following structural formula:

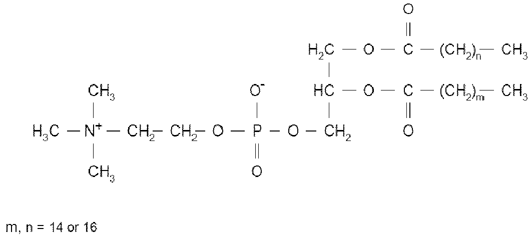

### INDICATIONS AND USAGE

Doxil® (doxorubicin HCl liposome injection) is indicated for the treatment of metastatic carcinoma of the ovary in patients with disease that is refractory to both paclitaxel- and platinum-based chemotherapy regimens. Refractory disease is defined as disease that has progressed while on treatment, or within 6 months of completing treatment.

These indications are based on objective tumor response rates. No results are available from controlled trials that demonstrate a clinical benefit resulting from this treatment, such as improvement in disease-related symptoms or increased survival.

### DOSAGE AND ADMINISTRATION

Ovarian Cancer Patients: Doxil® (doxorubicin HCl liposome injection) should be administered intravenously at a dose of 50 mg/m2 (doxorubicin HCl equivalent) at an initial rate of 1 mg/min to minimize the risk of infusion reactions. If no infusion-related AEs are observed, the rate of infusion can be increased to complete administration of the drug over one hour. The patient should be dosed once every 4 weeks, for as long as the patient does not progress, shows no evidence of cardiotoxicity, and continues to tolerate treatment. A minimum of 4 courses is recommended because median time to response in clinical trials was 4 months. To manage adverse events such as PPE, stomatitis or hematologic toxicity the doses may be delayed or reduced (see Dose Modification guidelines below). Pretreatment with or concomitant use of antiemetics should be considered.

Do not administer as a bolus injection or an undiluted solution. Rapid infusion may increase the risk of infusion-related reactions.

Each vial contains 20 mg doxorubicin HCl at a concentration of 2 mg/mL.

Until specific compatibility data are available, it is not recommended that Doxil® be mixed with other drugs.

Doxil® should be considered an irritant and precautions should be taken to avoid extravasation. With intravenous administration of Doxil®, extravasation may occur with or without an accompanying stinging or burning sensation, even if blood returns well on aspiration of the infusion needle. If any signs or symptoms of extravasation have occurred the infusion should be immediately terminated and restarted in another vein. The application of ice over the site of extravasation for approximately 30 minutes may be helpful in alleviating the local reaction. Doxil® must not be given by the intramuscular or subcutaneous route.

Dose Modification Guidelines: Doxil® exhibits nonlinear pharmacokinetics at 50 mg/m2; therefore, dose adjustments may result in a non-proportional greater change in plasma concentration and exposure to the drug.

Patients should be carefully monitored for toxicity. Adverse events, such as PPE, hematologic toxicities, and stomatitis may be managed by dose delays and adjustments. Following the first appearance of a Grade 2 or higher adverse event, the dosing should be adjusted or delayed as described in the following tables. Once the dose has been reduced, it should not be increased at a later time.

**Recommended Dose Modification Guidelines:**

| **PALMAR - PLANTAR ERYTHRODYSESTHESIA** | |
|---|---|
| **Toxicity Grade** | **Dose Adjustment** |
| | |
| **1** | |
| (mild erythema, swelling, or desquamation not interfering with daily activities) | **Redose unless patient has experienced previous Grade 3 or 4** toxicity. If so, delay up to 2 weeks and decrease dose by 25%. Return to original dose interval. |
| **2** | |
| (erythema, desquamation, or swelling interfering with, but not precluding normal physical activities; small blisters or ulcerations less than 2 cm in diam.) | **Delay dosing up to 2 weeks or until resolved to Grade 0-1.** If after 2 weeks there is no resolution, Doxil® should be discontinued. |
| **3** | |
| (blistering, ulceration, or swelling interfering with walking or normal daily activities; cannot wear regular clothing) | **Delay dosing up to 2 weeks or until resolved to Grade 0-1.** Decrease dose by 25% and return to original dose interval. If after 2 weeks there is no resolution, Doxil^{®} should be discontinued. |
| **4** | |
| (diffuse or local process causing infectious complications, or a bed ridden state or hospitalization) | **Delay dosing up to 2 weeks or until resolved to Grade 0-1.** Decrease dose by 25% and return to original dose interval. If after 2 weeks there is no resolution, Doxil® should be discontinued. |

| **HEMATOLOGICAL TOXICITY** | | | |
|---|---|---|---|
| **GRADE** | **ANC** | **PLATELETS** | **MODIFICATION** |
| **1** | 1500 - 1900 | 75,000 - 150,000 | Resume treatment with no dose reduction |
| **2** | 1000 - <1500 | 50,000 - <75,000 | Wait until ANC ≥ 1,500 and platelets ≥ 75,000; redose with no dose reduction |
| **3** | 500 - 999 | 25,000 - <50,000 | Wait until ANC ≥ 1,500 and platelets ≥ 75,000; redose with no dose reduction |
| | | | |
| **4** | <500 | <25,000 | Wait until ANC ≥ 1,500 and platelets ≥ 75,000; redose at 25% dose reduction or continue full dose with cytokine support. |

| **STOMATITIS** | |
|---|---|
| **Toxicity Grade** | **Dose Adjustment** |
| **1** | |
| (painless ulcers erythema, or mild soreness) | **Redose unless patient has experienced previous Grade 3 or 4** toxicity. If so, delay up to 2 weeks and decrease dose by 25%. Return to original dose interval. |
| **2** | |
| (painful erythema, edema, or ulcers, but can eat) | **Delay dosing up to 2 weeks or until resolved to Grade 0-1.** If after 2 weeks there is no resolution Doxil^{®} should be discontinued. |
| **3** | |
| (painful erythema, edema, or ulcers, but cannot eat) | **Delay dosing up to 2 weeks or until resolved to Grade 0-1.** Decrease dose by 25% and return to original dose interval. If after 2 weeks there is no resolution, Doxil^{®} should be discontinued. |
| **4** | |
| (requires parenteral or enteral support) | **Delay dosing up to 2 weeks or until resolved to Grade 0-1.** Decrease dose by 25% and return to original dose interval. If after 2 weeks there is no resolution, Doxil^{®} should be discontinued. |

Patients with Impaired Hepatic Function: Limited clinical experience exists in treating hepatically impaired patients with Doxil®. Based on experience with doxorubicin HCl, it is recommended that Doxil® dosage be reduced if the bilirubin is elevated as follows: Serum biliribin 1.2 to 3.0 mg/dL give ½ normal dose, >3 mg/dL give ¼ normal dose.

Preparation for Intravenous Administration: The appropriate dose of Doxil®, up to a maximum of 90 mg, must be diluted in 250 mL of 5% Dextrose Injection, USP prior to administration. Aseptic technique must be strictly observed since no preservative or bacteriostatic agent is present in Doxil®. Diluted Doxil® should be refrigerated at 2°C to 8°C (36°F to 46°F) and administered within 24 hours.

### Gemzar (Gemcitabine Hydrochloride)

### DESCRIPTION

Gemzar (gemcitabine for injection, USP) is a nucleoside metabolic inhibitor that exhibits antitumor activity. Gemcitabine HCl is 2'-deoxy-2',2'-difluorocytidine monohydrochloride (-isomer).

The structural formula is as follows:

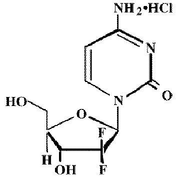

The empirical formula for gemcitabine HCl is C9H11F2N304 • HCl. It has a molecular weight of 299.66.

Gemcitabine HCl is a white to off-white solid. It is soluble in water, slightly soluble in methanol, and practically insoluble in ethanol and polar organic solvents.

The clinical formulation is supplied in a sterile form for intravenous use only. Vials of Gemzar contain either 200 mg or 1 g of gemcitabine HCl (expressed as free base) formulated with mannitol (200 mg or 1 g, respectively) and sodium acetate (12.5 mg or 62.5 mg, respectively) as a sterile lyophilized powder. Hydrochloric acid and/or sodium hydroxide may have been added for pH adjustment.

### INDICATIONS AND USAGE

Ovarian Cancer: Gemzar in combination with carboplatin is indicated for the treatment of patients with advanced ovarian cancer that has relapsed at least 6 months after completion of platinum-based therapy.

### DOSAGE AND ADMINISTRATION

Gemzar is for intravenous use only. Gemzar may be administered on an outpatient basis.

Ovarian Cancer: Gemzar should be administered intravenously at a dose of 1000 mg/m2 over 30 minutes on Days 1 and 8 of each 21-day cycle. Carboplatin AUC 4 should be administered intravenously on Day 1 after Gemzar administration. Patients should be monitored prior to each dose with a complete blood count, including differential counts. Patients should have an absolute granulocyte count ≥ 1500 x 106/L and a platelet count ≥ 100,000 x 106/L prior to each cycle.

Dose Modifications: Gemzar dosage adjustment for hematological toxicity within a cycle of treatment is based on the granulocyte and platelet counts taken on Day 8 of therapy. If marrow suppression is detected, Gemzar dosage should be modified according to guidelines in Table 1.

**Table 1: Day 8 Dosage Reduction Guidelines for Gemzar in Combination with Carboplatin**

| Absolute granulocyte count (x 10⁶/L) | | Platelet count (x 10⁶/L) | % of full dose |
|---|---|---|---|
| ≥1500 | And | ≥100,000 | 100 |
| 1000-1499 | And/Or | 75,000-99,999 | 50 |
| <1000 | And/Or | <75,000 | Hold |

In general, for severe (Grade 3 or 4) non-hematological toxicity, except nausea/vomiting, therapy with Gemzar should be held or decreased by 50% depending on the judgment of the treating physician. For carboplatin dosage adjustment, see manufacturer's prescribing information.

Dose adjustment for Gemzar in combination with carboplatin for subsequent cycles is based upon observed toxicity. The dose of Gemzar in subsequent cycles should be reduced to 800 mg/m2 on Days 1 and 8 in case of any of the following hematologic toxicities:
- Absolute granulocyte count <500 x 106/L for more than 5 days
- Absolute granulocyte count <100 x 106/L for more than 3 days
- Febrile neutropenia
- Platelets <25,000 x 106/L
- Cycle delay of more than one week due to toxicity

If any of the above toxicities recur after the initial dose reduction, for the subsequent cycle, Gemzar should be given on Day 1 only at 800 mg/m2.

### Hycamtin (Topotecan Hydrochloride)

### DESCRIPTION

HYCAMTIN (topotecan hydrochloride) is a semi-synthetic derivative of camptothecin and is an anti-tumor drug with topoisomerase I-inhibitory activity.

HYCAMTIN for Injection is supplied as a sterile lyophilized, buffered, light yellow to greenish powder available in single-dose vials. Each vial contains topotecan hydrochloride equivalent to 4 mg of topotecan as free base. The reconstituted solution ranges in color from yellow to yellow-green and is intended for administration by intravenous infusion.

Inactive ingredients are mannitol, 48 mg, and tartaric acid, 20 mg. Hydrochloric acid and sodium hydroxide may be used to adjust the pH. The solution pH ranges from 2.5 to 3.5.

The chemical name for topotecan hydrochloride is (S)-10-[(dimethylamino)methyl]-4-ethyl-4,9-dihydroxy-1H-pyrano[3',4':6,7] indolizino [1,2-b]quinoline-3,14-(4H,12H)-dione monohydrochloride. It has the molecular formula C23H23N3O5•HCl and a molecular weight of 457.9.

Topotecan hydrochloride has the following structural formula:

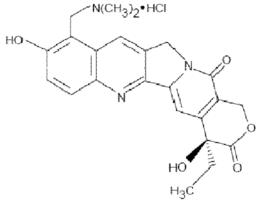

It is soluble in water and melts with decomposition at 213° to 218°C.

### INDICATIONS AND USAGE

HYCAMTIN is indicated for the treatment of metastatic carcinoma of the ovary after failure of initial or subsequent chemotherapy.

### DOSAGE AND ADMINISTRATION

Prior to administration of the first course of HYCAMTIN, patients must have a baseline neutrophil count of >1,500 cells/mm3 and a platelet count of >100,000 cells/mm3. The recommended dose of HYCAMTIN is 1.5 mg/m2 by intravenous infusion over 30 minutes daily for 5 consecutive days, starting on day 1 of a 21-day course. In the absence of tumor progression, a minimum of 4 courses is recommended because tumor response may be delayed. The median time to response in 3 ovarian clinical trials was 9 to 12 weeks, and median time to response in 4 small cell lung cancer trials was 5 to 7 weeks. In the event of severe neutropenia during any course, the dose should be reduced by 0.25 mg/m2 for subsequent courses. Doses should be similarly reduced if the platelet count falls below 25,000 cells/mm3. Alternatively, in the event of severe neutropenia, G-CSF may be administered following the subsequent course (before resorting to dose reduction) starting from day 6 of the course (24 hours after completion of topotecan administration).

Adjustment of Dose in Special Populations: Hepatic Impairment: No dosage adjustment appears to be required for treating patients with impaired hepatic function (plasma bilirubin >1.5 to <10 mg/dL).

Renal Functional Impairment: No dosage adjustment appears to be required for treating patients with mild renal impairment (Clcr 40 to 60 mL/min.). Dosage adjustment to 0.75 mg/m2 is recommended for patients with moderate renal impairment (20 to 39 mL/min.). Insufficient data are available in patients with severe renal impairment to provide a dosage recommendation.

Elderly Patients: No dosage adjustment appears to be needed in the elderly other than adjustments related to renal function.

### TAXOL (paclitaxel)

### DESCRIPTION

TAXOL (paclitaxel) Injection is a clear, colorless to slightly yellow viscous solution. It is supplied as a nonaqueous solution intended for dilution with a suitable parenteral fluid prior to intravenous infusion. TAXOL is available in 30 mg (5 mL), 100 mg (16.7 mL), and 300 mg (50 mL) multidose vials. Each mL of sterile nonpyrogenic solution contains 6 mg paclitaxel, 527 mg of purified Cremophor® EL (polyoxyethylated castor oil) and 49.7% (v/v) dehydrated alcohol, USP.

Paclitaxel is a natural product with antitumor activity. TAXOL (paclitaxel) is obtained via a semi-synthetic process from Taxus baccata. The chemical name for paclitaxel is 5β,20-Epoxy-1,2α,4,7β,10β,13α-hexahydroxytax-11-en-9-one 4,10-diacetate 2-benzoate 13-ester with (2R,3S)-N-benzoyl-3-phenylisoserine.

Paclitaxel has the following structural formula:

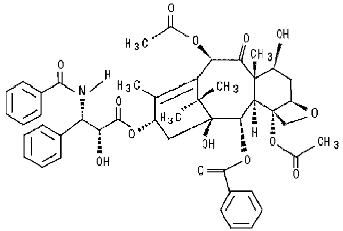

Paclitaxel is a white to off-white crystalline powder with the empirical formula C47H51NO14 and a molecular weight of 853.9. It is highly lipophilic, insoluble in water, and melts at around 216-217° C.

### INDICATIONS AND USAGE

TAXOL is indicated as first-line and subsequent therapy for the treatment of advanced carcinoma of the ovary. As first-line therapy, TAXOL is indicated in combination with cisplatin.

### DOSAGE AND ADMINISTRATION

For patients with carcinoma of the ovary, the following regimens are recommended:
1) For previously untreated patients with carcinoma of the ovary, one of the following recommended regimens may be given every 3 weeks. In selecting the appropriate regimen, differences in toxicities should be considered.
   a. TAXOL administered intravenously over 3 hours at a dose of 175 mg/m2 followed by cisplatin at a dose of 75 mg/m2; or
   b. TAXOL administered intravenously over 24 hours at a dose of 135 mg/m2 followed by cisplatin at a dose of 75 mg/m2.
2) In patients previously treated with chemotherapy for carcinoma of the ovary, TAXOL has been used at several doses and schedules; however, the optimal regimen is not yet clear. The recommended regimen is TAXOL 135 mg/m2 or 175 mg/m2 administered intravenously over 3 hours every 3 weeks.

### Paraplatin (Carboplatin)

### DESCRIPTION

PARAPLATIN® (carboplatin) for Injection, USP is supplied as a sterile, lyophilized white powder available in single-dose vials containing 50 mg, 150 mg, and 450 mg of carboplatin for administration by intravenous infusion. Each vial contains equal parts by weight of carboplatin and mannitol.

Carboplatin is a platinum coordination compound that is used as a cancer chemotherapeutic agent. The chemical name for carboplatin is platinum, diammine[1,1-cyclobutanedicarboxylato(2-)-O,O']-, (SP-4-2), and has the following structural formula:

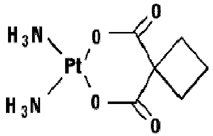

Carboplatin is a crystalline powder with the molecular formula of C6H12N2O4Pt and a molecular weight of 371.25. It is soluble in water at a rate of approximately 14 mg/mL, and the pH of a 1% solution is 5 to 7. It is virtually insoluble in ethanol, acetone, and dimethylacetamide.

### INDICATIONS AND USAGE

### Initial Treatment of Advanced Ovarian Carcinoma:

PARAPLATIN (carboplatin) for Injection, USP is indicated for the initial treatment of advanced ovarian carcinoma in established combination with other approved chemotherapeutic agents. One established combination regimen consists of PARAPLATIN and cyclophosphamide. Two randomized controlled studies conducted by the NCIC and SWOG with PARAPLATIN versus cisplatin, both in combination with cyclophosphamide, have demonstrated equivalent overall survival between the two groups.

There is limited statistical power to demonstrate equivalence in overall pathologic complete response rates and long-term survival (≥3 years) because of the small number of patients with these outcomes: the small number of patients with residual tumor <2 cm after initial surgery also limits the statistical power to demonstrate equivalence in this subgroup.

### Secondary Treatment of Advanced Ovarian Carcinoma

PARAPLATIN is indicated for the palliative treatment of patients with ovarian carcinoma recurrent after prior chemotherapy, including patients who have been previously treated with cisplatin.

Within the group of patients previously treated with cisplatin, those who have developed progressive disease while receiving cisplatin therapy may have a decreased response rate.

### DOSAGE AND ADMINISTRATION

### Single-Agent Therapy

PARAPLATIN (carboplatin) for Injection, USP, as a single agent, has been shown to be effective in patients with recurrent ovarian carcinoma at a dosage of 360 mg/m2 IV on day 1 every 4 weeks. In general, however, single intermittent courses of PARAPLATIN should not be repeated until the neutrophil count is at least 2,000 and the platelet count is at least 100,000.

### Combination Therapy with Cyclophosphamide

In the chemotherapy of advanced ovarian cancer, an effective combination for previously untreated patients consists of :

PARAPLATIN-300 mg/m2 IV on day 1 every 4 weeks for 6 cycles (alternatively see Formula Dosing).

Cyclophosphamide-600 mg/m2 IV on day 1 every 4 weeks for 6 cycles. For directions regarding the use and administration of cyclophosphamide please refer to its package insert.

Intermittent courses of PARAPLATIN in combination with cyclophosphamide should not be repeated until the neutrophil count is at least 2,000 and the platelet count is at least 100,000.

### Dose Adjustment Recommendations

Pretreatment platelet count and performance status are important prognostic factors for severity of myelosuppression in previously treated patients.

The suggested dose adjustments for single agent or combination therapy shown in the table below are modified from controlled trials in previously treated and untreated patients with ovarian carcinoma. Blood counts were done weekly, and the recommendations are based on the lowest post-treatment platelet or neutrophil value.

| **Platelets** | **Neutrophils** | **Adjusted Dose* (From Prior Course)** |
|---|---|---|
| > 100,000 | >2,000 | 125% |
| 50-100,000 | 500-2,000 | No Adjustment |
| <50,000 | <500 | 75% |

| | | |
|---|---|---|
| * Percentages apply to PARAPLATIN (carboplatin) for Injection. USP as a single agent or to both PARAPLATIN and cyclophosphamide in combination. In the controlled studies, dosages were also adjusted at a lower level (50% to 60%) for severe myelosuppression. Escalations above 125% were not recommended for these studies. | | |

PARAPLATIN is usually administered by an infusion lasting 15 minutes or longer. No pre- or post-treatment hydration or forced diuresis is required.

### Patients with Impaired Kidney Function

Patients with creatinine clearance values below 60 mL/min are at increased risk of severe bone marrow suppression. In renally-impaired patients who received single-agent PARAPLATIN therapy, the incidence of severe leukopenia, neutropenia, or thrombocytopenia has been about 25% when the dosage modifications in the table below have been used.

| **Baseline Creatinine Clearance** | **Recommended Dose on Day 1** |
|---|---|
| 41 mL/min - 59 mL/min | 250 mg/m² |
| 16 mL/min - 40 mL/min | 200 mg/m² |

The data available for patients with severely impaired kidney function (creatinine clearance below 15 mL/min) are too limited to permit a recommendation for treatment.

These dosing recommendations apply to the initial course of treatment. Subsequent dosages should be adjusted according to the patient's tolerance based on the degree of bone marrow suppression.

### Formula Dosing

Another approach for determining the initial dose of PARAPLATIN is the use of mathematical formulae, which are based on a patient's pre-existing renal function or renal function and desired platelet nadir. Renal excretion is the major route of elimination for carboplatin. The use of dosing formulae, as compared to empirical dose calculation based on body surface area, allows compensation for patient variations in pretreatment renal function that might otherwise result in either underdosing (in patients with above average renal function) or overdosing (in patients with impaired renal function).

A simple formula for calculating dosage, based upon a patient's glomerular filtration rate (GFR in mL/min) and PARAPLATIN target area under the concentration versus time curve (AUC in mg/mL•min), has been proposed by Calvert. In these studies, GFR was measured by 51Cr-EDTA clearance.

| CALVERT FORMULA FOR CARBOPLATIN DOSING |
|---|
| Total Dose (mg)=(target (AUC) × (GFR + 25) |
| **Note: With the Calvert formula, the total dose of PARAPLATIN is calculated in mg, not mg/m².** |

The target AUC of 4 mg/mL•min to 6 mg/mL•min using single-agent PARAPLATIN appears to provide the most appropriate dose range in previously treated patients. This study also showed a trend between the AUC of single-agent PARAPLATIN administered to previously treated patients and the likelihood of developing toxicity.

| | % Actual Toxicity in Previously Treated Patients | |
|---|---|---|
| AUC (mg/mL•min) | Gr 3 or Gr 4 Thrombocytopenia | Gr 3 or Gr 4 Leukopenia |
| 4 to 5 | 16% | 13% |
| 6 to 7 | 33% | 34% |

### Geriatric Dosing

Because renal function is often decreased in elderly patients, formula dosing of PARAPLATIN based on estimates of GFR should be used in elderly patients to provide predictable plasma PARAPLATIN AUCs and thereby minimize the risk of toxicity.

### Platinol (Cisplatin)

### DESCRIPTION

PLATINOL® (cisplatin for injection, USP) is a white to light yellow lyophilized powder. Each vial of PLATINOL contains 50 mg cisplatin, 450 mg Sodium Chloride, USP, and 500 mg Mannitol, USP.

The active ingredient, cisplatin, is a yellow to orange crystalline powder with the molecular formula PtCl2H6N2, and a molecular weight of 300.1. Cisplatin is a heavy metal complex containing a central atom of platinum surrounded by two chloride atoms

and two ammonia molecules in the cis position. It is soluble in water or saline at 1 mg/mL and in dimethylformamide at 24 mg/mL. It has a melting point of 207° C.

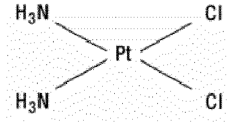

### INDICATIONS AND USAGE

PLATINOL (cisplatin for injection, USP) is indicated as therapy to be employed as follows:
Metastatic Ovarian Tumors

In established combination therapy with other approved chemotherapeutic agents in patients with metastatic ovarian tumors who have already received appropriate surgical and/or radiotherapeutic procedures. An established combination consists of PLATINOL and cyclophosphamide. PLATINOL, as a single agent, is indicated as secondary therapy in patients with metastatic ovarian tumors refractory to standard chemotherapy who have not previously received PLATINOL therapy.

### DOSAGE AND ADMINISTRATION

The usual PLATINOL dose for the treatment of metastatic ovarian tumors in combination with cyclophosphamide is 75 to 100 mg/m2 IV per cycle once every 4 weeks (DAY 1).

The dose of cyclophosphamide when used in combination with PLATINOL is 600 mg/m2 IV once every 4 weeks (DAY 1).

For directions for the administration of cyclophosphamide, refer to the cyclophosphamide package insert.

In combination therapy, PLATINOL and cyclophosphamide are administered sequentially.

As a single agent, PLATINOL should be administered at a dose of 100 mg/m2 IV per cycle once every 4 weeks.

Pretreatment hydration with 1 to 2 liters of fluid infused for 8 to 12 hours prior to a PLATINOL dose is recommended. The drug is then diluted in 2 liters of 5% Dextrose in 1/2 or 1/3 normal saline containing 37.5 g of mannitol, and infused over a 6- to 8-hour period. If diluted solution is not to be used within 6 hours, protect solution from light. Adequate hydration and urinary output must be maintained during the following 24 hours.

A repeat course of PLATINOL should not be given until the serum creatinine is below 1.5 mg/100 mL, and/or the BUN is below 25 mg/100 mL. A repeat course should not be given until circulating blood elements are at an acceptable level (platelets ≥100,000/mm3, WBC ≥4000/mm3). Subsequent doses of PLATINOL should not be given until an audiometric analysis indicates that auditory acuity is within normal limits.

Although the present invention has been described with reference to particular examples and embodiments, it should be understood that the present invention is not limited to those examples and embodiments. Moreover, the features of the particular examples and embodiments may be used in any combination. The present invention therefore includes variations from the various examples and embodiments described herein, as will be apparent to one of skill in the art.

### Equivalents

Those skilled in the art will recognize, or be able to ascertain using no more than routine experimentation, many equivalents to the specific embodiments of the invention described herein. The scope of the present invention is not intended to be limited to the above Description, but rather is as set forth in the following claims:

## Claims

1. An antibody specific for CA-125, HE4, mesothelin, and/or YKL-40 for use in a method of diagnosing ovarian cancer, wherein the antibody is comprised in an implantable bio-sensing device, wherein the bio-sensing device comprises and/or is a transducer that is functionalized for detection of CA-125, HE4, mesothelin, and/or YKL-40, wherein the functionalization comprises association of the antibody specific for CA-125, HE4, mesothelin, and/or YKL-40 to the transducer.

2. The antibody for use of claim 1, wherein the antibody is coupled to the functionalized transducer so that the bio-sensing device creates a signal that can be transmitted from the transceiver layer to activate a remote sensor.

3. The antibody for use of claim 1 or 2, wherein the bio-sensing device comprises a microfluidics concentrator chamber, a transducer layer and a transceiver layer.

4. The antibody for use of claim 3, wherein the use comprises allowing body fluids to pass through the microfluidics concentrator chamber and detecting CA-125, HE4, mesothelin, and/or YKL-40 on the transducer layer.

5. The antibody for use of any one of the preceding claims, wherein the bio-sensing device is implanted in the subject's endometrial/uterine cavity.

6. The antibody for use of any one of the preceding claims, wherein
(a) the use comprises detecting CA-125, HE4, mesothelin, and/or YKL-40 in a uterine sample, intrauterine fluid or a uterine wash sample *in vivo;*
(b) the use comprises detecting CA-125, HE4, mesothelin, and/or YKL-40 in cells that are shed from the fallopian tube, wherein the cells have optionally been implanted in the ovary; and/or
(c) the device is implantable in a body cavity, wherein the cavity optionally has continuity with the fallopian tubes, wherein the cavity is optionally a uterine cavity.

7. The antibody for use of any one of the preceding claims, wherein the use comprises transmitting a signal from the bio-sensing device to a remote sensor, wherein the signal corresponds to data obtained during the step of measuring.

8. A method of determining the *in vivo* level of one or more biomarker selected from the group consisting of CA-125, HE4, mesothelin, and YKL-40 comprising detecting a signal transmitted from a bio-sensing device implanted in a subject using a remote sensor, wherein the signal is indicative for the level of the biomarker, wherein the level of the biomarker is measured by the implanted bio-sensing device *in vivo,* wherein the signal corresponds to data obtained during the step of measuring.

9. A method of emitting a signal that is indicative for the level of one or more biomarker selected from the group consisting of CA-125, HE4, mesothelin, and YKL-40, wherein the method comprises measuring the biomarker level via a bio-sensing device implanted *in vivo,* and emitting a signal from the bio-sensing device, wherein the signal corresponds to data obtained during the step of measuring.

10. The method of claim 9, wherein the method comprises transmitting a signal from the bio-sensing device to a remote sensor.

11. The method of any one of claims 8 to 10, wherein
(a) the biomarker level is detected in a uterine sample, intrauterine fluid, or a uterine wash sample;
(b) the bio-sensing device is implanted in the subject's endometrial/uterine cavity;
(c) the method comprises detecting CA-125, HE4, mesothelin, and/or YKL-40 in cells that are shed from the fallopian tube, wherein the cells have optionally been implanted in the ovary; and/or
(d) the device is implantable in a body cavity, wherein the cavity optionally has continuity with the fallopian tubes, wherein the cavity is optionally a uterine cavity.

12. The method of any one of claims 8 to 11, wherein
(a) the signal is useful for the detection and/or characterization of ovarian cancer; and/or
(b) the bio-sensing device comprises a construct that comprises single or multi-walled carbon nanotubes to which antibodies to the CA-125, HE4, mesothelin, and/or YKL-40 are coupled.

13. The antibody for use of any one of claims 1 to 7 or the method of any one of claims 8 to 12, wherein the ovarian cancer is high grade serous carcinoma.

14. A bio-sensing device that is configured to intermittently and frequently monitor body tissues for CA-125, HE4, mesothelin, and/or YKL-40, wherein the device is implantable into a subject's endometrial/uterine cavity, wherein the device includes a microfluidics concentrator chamber, a transducer layer and a transceiver layer, wherein body fluids are allowed to pass through the microfluidics concentrator chamber, wherein the transducer layer is functionalized through association to an antibody comprising anti-CA-125 antibody, anti-HE4 antibody, anti-mesothelin antibody, anti-YKL-40 antibody, wherein the bio-sensing device is capable of creating a signal that can be transmitted from the transceiver layer to activate a remote sensor.

15. The device of claim 14, wherein
(a) the device is configured to detect and/or monitor CA-125, HE4, mesothelin, and/or YKL-40 in cells that are shed from the fallopian tube, wherein the cells have optionally been implanted in the ovary; and/or
(b) the device is implantable in a body cavity, wherein the cavity optionally has continuity with the fallopian tubes, wherein the cavity is optionally a uterine cavity.
